# EUROPÄISCHE PATENTSCHRIFT

(11) **EP 0 716 103 B1**
(45) Veröffentlichungstag und Bekanntmachung des Hinweises auf die Patenterteilung: **12.05.1999**
(21) Anmeldenummer: 95250279.7
(22) Anmeldetag: 17.11.1995
(51) Int. Cl.: C08F 283/00, C08F 291/00, A61K 6/083

(54) **Feinkörnige unter Druck oder Scherbeanspruchung fliessfähige polymerisierbare Zusammensetzungen**
Polymerisable powder composition, flowable under pressure or under shearing
Compositions polymérisables de poudre fine, fluide sous pression ou soumise à une force de cisaillement

(30) Priorität: 08.12.1994 DE 4443702
(43) Veröffentlichungstag der Anmeldung: 12.06.1996
(73) Patentinhaber: IVOCLAR AG, FL-9494 Schaan (LI)
(72) Erfinder: Rheinberger, Volker, FL-9490 Vaduz (LI); Moszner, Norbert, FL-9492 Eschen (LI); Völkel, Thomas, D-88131 Lindau (DE); Burtscher, Peter, A-6714 Nütziders (AT)
(74) Vertreter: UEXKÜLL & STOLBERG

(56) Entgegenhaltungen:
- EP-A- 0 449 399
- EP-A- 0 576 112
- WO-A-93/14147
- US-A- 4 568 737

## Beschreibung

Die vorliegende Erfindung betrifft hochgefüllte, körnige, dendrimerhaltige, polymerisierbare Zusammensetzungen, die bei Druck- und/oder Scherbeanspruchung fließfähig werden und damit formbar sind. Die erfindungsgemäßen Zusammensetzungen eignen sich besonders als Dentalmaterialien, Komponente von Dentalmaterialien oder zur Herstellung von Dentalmaterialien.

Polymerisierbare Zusammensetzungen werden auf vielen Gebieten eingesetzt, u.a. auch als Dentalmassen sowie als Kleb- und Spachtelmassen. Im Hinblick auf die Haltbarkeit der ausgehärteten Zusammensetzung wird dabei im allgemeinen ein möglichst hoher Füllstoffgehalt angestrebt. Polymerisierbare Zusammensetzungen mit hohen Füllstoffgehalten, die sich als Dentalmaterialien eignen, werden beispielsweise in der *International Encyclopedia of Composites* (S.M. Lee, Herausgeber, Vol. 2, VCH-Verlagsgesellschaft, New York 1990, Seite 182) und von L. Ehrnford (Swed. Dent. J., Suppl. 18, 1983) beschrieben. Andererseits sind hochgefüllte Massen nur noch schwierig plastisch verformbar, was ihre Weiterverarbeitung erschwert und den maximalen Füllstoffgehalt begrenzt. Aus der EP-A-0 480 472 sind härtbare Polymermassen bekannt, die so hoch gefüllt sind, daß sie sich nur unter Einwirkung von Ultraschall-Schwingungen verarbeiten lassen.

EP- A- 0 576 112 beschreibt ein Dentalmaterial aus einer polymerisierbaren Zusammensetzung, die mindestens ein ethylenisch ungesättigtes Monomer, einen Polymerisationsinitiator, einen Beschleuniger, 20- 80 Gew.% Füllstoff und ein polyfunktionelles Epimin (Aziridin) enthält. Polyfunktionelle Epimine sind beispielsweise Verbindungen, bei denen an den Seitenketten oder Enden von im wesentlichen linearen Polyethern, Polythioethern oder gesättigten Polyestern Ethyleniminreste eingeführt sind. Das Dentalmaterial durchläuft bei der Verarbeitung eine erste elastische Phase, in der das Material mechanisch bearbeitet oder Überschüsse entfernt werden und eine zweite ausgehärtete Stufe.

Die WO-A-93/17060 betrifft dendritische Makromoleküle auf der Basis von Polyestern, die mehr durch eine hochverzweigte (hyper-branched) Struktur statt einer ideal verzweigten Dendrimerstruktur charakterisiert sind, und Verfahren zu deren Herstellung. Die Dendrimere eignen sich u.a. als Komponente für polymerisierbare Zusammensetzungen, wobei jedoch lediglich flüssige Lacke beschrieben werden, füllstoffhaltige Zusammensetzungen werden nicht offenbart.

Die WO-A-93/14147 betrifft dendrimere Makromoleküle, deren Äste aus Vinylcyanideinheiten aufgebaut sind, sowie Verfahren zu deren Herstellung. Diese Dendrimere eignen sich u.a. zum Mischen mit thermoplastischen Polymeren oder polymeren Zusammensetzungen. Dendrimere mit polymerisationsfähigen Gruppen oder hochgefüllte Mischungen werden nicht erwähnt.

Polymerisierbare Zusammensetzungen, die einen so hohen Füllstoffgehalt aufweisen, daß sie statt einer hochviskosen Konsistenz einen körnigen Aufbau zeigen und dennoch gut plastisch verformbar sind, sind bisher nicht bekannt.

Aufgabe der Erfindung ist die Schaffung einer polymerisierbaren Zusammensetzung mit körniger Konsistenz, die unter Druck- und/oder Scherbeanspruchung fließfähig und damit formbar wird und die sich besonders zur Anwendung als Dentalmaterial, Komponente von Dentalmaterial oder zur Herstellung eines Dentalmaterials eignet.

Diese Aufgabe wird überraschenderweise durch eine polymerisierbare Zusammensetzung gelöst, die neben mindestens einem polymerisierbarem Monomer und/oder Oligomer sowie mindestens einem Füllstoff und einem Polymerisationsinitiator und ggf. einem Beschleuniger 0,5 bis 28 Gew.% eines Dendrimers enthält. Der Füllstoffgehalt der erfindungsgemäßen Zusammensetzungen beträgt mindestens 70 Gew.-%. Diese Zusammensetzungen weisen eine körnige Konsistenz auf; sie werden jedoch bei Druck- und/oder Scherbeanspruchung fließfähig und formbar.

Als Dendrimere werden dreidimensionale, hochgeordnete oligomere und polymere Verbindungen bezeichnet, die ausgehend von kleinen Initiator-Molekülen durch eine sich ständig wiederholende Reaktionsfolge synthetisiert werden. Als Initiator eignen sich monomere oder polymere Moleküle mit mindestens einer reaktiven Stelle. Diese wird in einer ein- oder mehrstufigen Reaktion mit einem Reaktanden umgesetzt, der sich an die reaktive Stelle des Initiators addiert und der seinerseits mindestens zwei neue reaktive Stellen zur Verfügung stellt. Die Umsetzung von Initiator und Reaktand ergibt die Kern-Zelle (Generation Null). Durch eine Wiederholung der Reaktion werden die reaktiven Stellen der ersten Reaktandenschicht mit weiteren Reaktanden umgesetzt, wobei wieder jeweils mindestens zwei neue Verzweigungszentren in das Molekül eingebracht werden (1. Generation). Die fortschreitende Verzweigung führt zu einem geometrischen Wachstum der Anzahl der Atome für jede Generation. Da die Gesamtgröße wegen der durch die Reaktanden festgelegten Zahl der möglichen kovalenten Bindungen nur linear wachsen kann, werden die Moleküle von Generation zu Generation gedrängter, und sie verändern ihre Form von seesternartig zu kugelig. Es ist möglich, derartige Dendrimere bis zu einer sich selbst begrenzenden Größe ("self-limiting generation") wachsen zu lassen. Die selbstbegrenzende Größe wird durch die Zahl der reaktiven Stellen des Initiators und der Repetiereinheiten sowie die Abmessungen der einzelnen Bausteine festgelegt. Durch die Wahl dieser Parameter ist es möglich, Größe, Gestalt, Topologie, Flexibilität und Oberflächenchemie der Dendrimere zu kontrollieren. Eine hohe Initiatorkernmultiplizität, eine hohe Verzweigungszentrenmultiplizität und kurze Verzweigungssegmentlängen ergeben sehr kompakte Makromoleküle mit kleinen Hohlräumen, während umgekehrt eine niedrige Initiatorkernmultiplizität, eine niedrige Verzweigungszentrenmultiplizität und eine lange Segmentlänge zur Ausbildung großer Hohlräume führt. Bei gegebener Initiatorkernmultiplizität, Verzweigungszentrenmultiplizität und Verzweigungssegmentlänge nimmt die innere Oberfläche mit der Generationszahl zu.

Erfindungsgemäß bevorzugte Dendrimere werden durch Reaktion von hydroxyl- oder aminogruppenhaltigen Initiatormolekülen mit Vinylcyaniden, wie beispielsweise Acryl- oder Methacrylnitril erhalten (Propylenimin-Dendrimere). Geeignete Propylenimin-Dendrimere und Verfahren zu deren Herstellung werden in der WO-A-93/14147 beschrieben. Weitere Gruppen bevorzugter Dendrimere stellen die Polyether/Polythioether- (A.B. Padias et al.; Polym. Prepr. Am. Chem. Soc., Div. Polym. Chem. **30** (1989) 119), die Polyester- (WO-A-93/17060), die Polyphenylenamid- (S.C.E. Backson et al.; Macromol. Symp. **77** (1994) 1) sowie die Polyphenylenester-Dendrimere (K.L. Wooley et al., Polymer Journal **26** (1994) 187) dar. Mischungen der genannten Dendrimere sind ebenfalls einsetzbar.

Weiterhin sind Dendrimere bevorzugt, die eine sphärische Struktur aufweisen. Zudem sind Dendrimere der 4. oder einer höheren Generation erfindungsgemäß besonders bevorzugt.

In einer bevorzugten Ausführungsform weisen die Dendrimere polymerisationsfähige Endgruppen auf. Als Endgruppen werden die reaktiven Gruppen der letzten Reaktandengeneration bezeichnet. Bevorzugte polymerisierbare Gruppen sind (Meth)acryl-, Allyl-, Styryl-, Vinyl-, Vinyloxy- und/oder Vinylamin-Gruppen.

Die Synthese von Dendrimeren mit polymerisationsfähigen Endgruppen erfolgt durch die aus der organischen Chemie bekannten Umsetzungen der o.g. Dendrimere mit geeigneten Monomer-Reagentien. Besonders geeingete Ausgangsmaterialien sind Dendrimere mit Carboxyl-, Hydroxyl- und/oder Aminoendgruppen. Methacrylsäurechlorid und Isocyanatoethylmethacrylat sind zur Umsetzung hydroxy- oder aminofunktionalisierter Dendrimere und 2-Hydroxyethylmethacrylat zur Umsetzung carboxy gruppenhaltiger Dendrimere bevorzugt. Zur Umsetzung von aminogruppenhaltigen Dendrimeren ist die Michael-Reaktion mit Acryloyloxyethylmethacrylat (AEMA) besonders bevorzugt. Die Michael-Reaktion erfolgt selektiv an der Acrylat-Doppelbindung, während die Methacrylat-Doppelbindung als polymerisationsfähige Gruppe erhalten bleibt.

Die Herstellung der erfindungsgemäßen polymerisierbaren Zusammensetzungen erfolgt durch Mischen des Dendrimers mit mindestens einem polymerisierbaren Monomer und/oder Oligomer. Anschließend wird diese Mischung portionsweise mit einem Füllstoff versetzt, bis sie eine pastenartige Konsistenz aufweist. Zusätzlich wird der Mischung ein Polymerisationsinitiator, vorzugsweise ein Photoinitiator, sowie ggf. ein Beschleuniger zugesetzt.

Als polymerisierbares Monomer und/oder Oligomer werden vorzugsweise mono- oder polyfunktionelle Methacrylate verwendet. Besonders bevorzugte Monomere sind Methylmethacrylat, Triethylenglycoldimethacrylat, Hexandiolmethacrylat, Dodecandioldimethacrylat, Bisphenol-A-dimethacrylat, Bisphenol-A-glycidyldimethacrylat, Trimethylolpropantrimethacrylat und 2-Hydroxyethylmethacrylat sowie Urethandimethacrylate, d.h. Umsetzungsprodukte von Isocyanaten, insbesondere Di- und/oder Triisocyanaten, mit hydroxylgruppenhaltigen Methacrylaten. Besonders bevorzugt sind Bisphenol-A-diglycidyldimethacrylat und das Urethandimethacrylat aus 2-Hydroxyethylmethacrylat und 2,2,4-Trimethylhexamethylendiisocyanat-1,6. Der Monomer- und/oder Oligomergehalt der erfindungsgemäßen Zusammensetzungen beträgt vorzugsweise 0,5 bis 28 Gew.%, besonders bevorzugt 5 bis 20 Gew.-%.

Als Füllstoffe eignen sich besonders amorphe Kieselsäuren, insbesondere pyrogene und gefällte Kieselsäure mit einer BET-Oberfläche von 30 bis 300 m²/g, vorzugsweise von 30 bis 100 m²/g (z.B. Aerosil 200, Ox 50; Degussa AG), oder Zinkoxid (ZnO).

Ein ganz besonders bevorzugter Füllstoff ist ein amorphes, kugelförmiges Material auf der Basis von Siliciumdioxid, das daneben noch mindestens ein Oxid eines Metalles der Gruppen I, II, III und IV des Periodensystems enthält. Bevorzugt wird Strontium- und/oder Zirkonoxid verwendet. Die durchschnittliche Primärteilchengröße liegt im Bereich von 0,1 bis 1,0 µm, insbesondere bei 0,15 bis 0,5 µm. Der Brechungsindex des Materials liegt zwischen 1,50 und 1,58, insbesondere zwischen 1,52 und 1,56. Ein besonders bevorzugter Wert ist 1,53 ± 0,01. Es sind auch Füllstoffmischungen möglich, insbesondere solche, die die obigen Parameter hinsichtlich Teilchengröße und Brechungsindex erfüllen. Füllstoffe dieses Typs sind in der DE-C-32 47 800 offenbart und werden im Rahmen dieser Erfindung als Sphärosile bezeichnet. Der Füllstoff kann auch gesintert als Mischung von Agglomeraten mit einer durchschnittlichen Teilchengröße von 1 bis 30 µm vorliegen.

Als röntgenopake Füllstoffe eignen sich besonders röntgenopake Gläser, Bariumsulfat und Ytterbiumfluorid. Mischungen aus verschieden Füllstoffen, insbesondere aus den genannten röntgenopaken und nicht-röntgenopaken Füllstoffen, sind ebenfalls geeignet. Besonders geeignete Füllstoffmischungen sind in der DE-A-40 29 230, Seite 5, Zeile 18 bis Seite 6, Zeile 27 beschrieben.

Vorzugsweise werden anorganische Füllstoffe in der üblichen Weise mit einem Silan, vorzugsweise mit 3-Methacryloyloxypropyltrimethoxysilan, silanisiert.

Der Füllstoffanteil der erfindungsgemäßen polymerisierbaren Zusammensetzungen kann bis zu 92 Gew.% betragen. Vorzugsweise liegt er im Bereich von 75 Gew.% bis 85 Gew.%.

Bevorzugte Initiatoren für die Photopolymerisation sind Benzophenon und Benzoin sowie deren Derivate. Darüber hinaus stellen auch α-Diketone geeignete Photoinitiatoren dar. Besonders bevorzugt sind 9,10-Phenanthrenchinon, Diacetyl- und 4,4'-Dichlorbenzil. Ganz besonders bevorzugt ist Campherchinon. α-Diketone werden bevorzugt in Kombination mit Beschleunigern, beispielsweise in Kombination mit einem Amin als Reduktionsmittel eingesetzt. Bevorzugte Amine sind Cyanoethylmethylanilin (CEMA), Dimethyl-aminoethylmethacrylat, Triethanolamin und N,N-Dimethyl-sym.-xylidin. Das Verhältnis von Photoinitiator zu Amin beträgt im allgemeinen 1:1. Am meisten bevorzugt ist die Verwendung von 0,3 Gew.-% Campherchinon und 0,5 Gew.-% CEMA, bezogen auf die Gesamtzusammensetzung, als Photoinitiatorsystem.

Als Initiatoren für die Kaltpolymerisation können Radikale liefernde Systeme, zum Beispiel Benzoyl- oder Laurylperoxid zusammen mit Aminen, vorzugsweise N,N-Dimethyl-sym.-xylidin oder N,N-Dimethyl-p-toluidin eingesetzt werden.

Die Funktion des Aminbeschleunigers kann vorzugsweise auch durch aminogruppenhaltige Dendrimere übernommen werden.

Eine besonders bevorzugte Zusammensetzungen enthält:

| Gew.-% | Komponente |
|---|---|
| 18,3 | Monomermischung aus: Bisphenol-A-glycidylmethacrylat (39,2 Gew.-%); Urethandimethacrylat aus 2-Hydroxyethylmetharylat und 2,2,4-Trimethylhexamethylendiisocyanat-1,6 (20 Gew.-%); Triethylenglycoldimethacrylat (20 Gew.-%); Dendrimer (20 Gew.-%); Campherchinon (0,3 Gew.-%); CEMA (0,5 Gew.-%); |
| 67,2 | silanisiertes, feindisperses Siliciumdioxid; |
| 14,5 | Ytterbiumfluorid |

Nach dem Mischen der Komponenten wird die Masse im Kneter durch Anlegen eines Vakuums entlüftet. Der Druck während des Entlüftens beträgt vorzugsweise 5 bis 20 mbar. Hierbei kommt es zur Ausbildung einer körnigen, trocken erscheinenden Masse, die jedoch bei Druck- oder Scherbeanspruchung wieder pastenartig und fließfähig wird und sich so bequem mit der Hand, ggf. unter Zuhilfenahme geeigneter Instrumente, wie z.B. einem Spatel, in Formen einpassen läßt, wo sie anschließend ausgehärtet wird. Die erfindungsgemäßen Zusammensetzungen erlauben so das vollständige und paßgenaue Ausfüllen auch kleiner Defekte und Lücken und eignen sich besonders als Dentalmaterialien oder Komponente von Dentalmaterialien, wie Zahnfüllmassen, oder zur Herstellung von Dentalmaterialien beispielsweise für Inlays/Onlays, Kronen, Brücken und Zähne.

Die Erniedrigung der Viskosität der erfindungsgemäßen Zusammensetzungen ist wahrscheinlich darauf zurückzuführen, daß die Dendrimere unter Druck- oder Scherbelastung das während des Entlüftens in die Dendrimerhohlräume aufgenommene Monomer und/oder Oligomer quasi wie ein molekularer Schwamm wieder abgeben, so daß die körnige Zusammensetzung zu einer homogenen formbaren Masse wird. Ein besonderer Vorzug der erfindungsgemäßen Zusammensetzungen ist darin zu sehen, daß sie sich ähnlich wie Amalgam stopfen lassen, wodurch ihre Handhabbarkeit ("handling") wesentlich vereinfacht wird.

Die Härtung der erfindungsgemäßen Zusammensetzung erfolgt vorzugsweise durch radikalische Polymerisation. Diese wird vorzugsweise thermisch oder photochemisch initiiert. Die Vernetzungsdichte und die Materialeigenschaften der gehärteten Masse können dabei durch die Struktur des verwendeten Dendrimeren und/oder durch die Art des verwendeten Monomeren variiert werden. So läßt sich beispielsweise durch die Verwendung von weniger flexiblen Monomeren und/ oder Dendrimeren der E-Modul der ausgehärteten Materialien erhöhen. Bei Einsatz von flexiblen Monomer- und/oder Dendrimerkomponenten werden Materialien mit höherer Dehnung, beispielsweise für provisorische Füllungen erhalten. Im folgenden wird die Erfindung anhand von Ausführungsbeispielen näher erläutert.

### Beispiele

### Beispiel 1:

### Modifizierung von DAB(PA)₃₂ mit AEMA

3,51 g (1 mmol) Dendrimer DAB(PA)₃₂ (Fa. DSM, Dendrimer auf Polyethyleniminbasis mit 32 Aminoendgruppen, Initiator: 1,4-Diaminobutan (DAB)) in 5 ml Methanol werden bei ca. 8°C unter Lichtausschluß tropfenweise mit 10,61 g (58 mmol) AEMA (vgl. J. Luchtenberg, H. Ritter, *Macromol. Rapid. Commun.* **15** (1994) 81) in 5 ml Methanol versetzt. Das Gemisch wird eine Stunde bei Raumtemperatur und 24 Stunden bei 60°C gerührt, und anschließend wird das Methanol im Vakuum abgedampft. Es werden 14,6 g (95% Ausbeute; diese und die folgenden Ausbeuten beziehen sich auf die Dendrimereinwaage) einer klaren Flüssigkeit erhalten.
¹H-NMR (ppm, CDCl₃, 90 MHz):
1,6 (m, CH₂CH₂CH₂), 1,9 (s,CH₃ Methacryl), 2,3 - 2,5 (CH₂-N), 2,7 (breites t, CH₂CH₂CO₂, 3,6 und 4,3,(O-CH₂CH₂-O), 5,6 und 6,1 (2s, =CH₂). 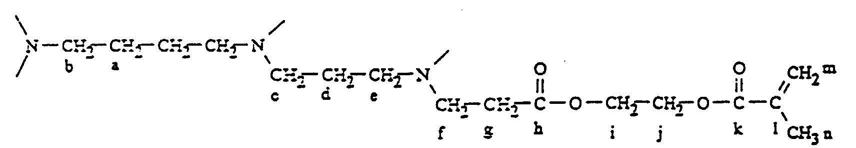 ¹³C-NMR(ppm, CDCl₃; 75 MHz;):
24,24 (a); 51,82 (b); 51,41 (c); 32,25 (d); 49,04 (e); 61,00 (f); 66,26 (g); 167,61 (h); 61,95 (i), 62,32 (j); 172,82 (k); 135,91 (l); 125,91 (m); 18,19 (n).
IR (Film, cm⁻¹):
2952 (C-H), 1724 (C=O).

Anhand von Konzentrationsreihen in Chloroform wird der Mark-Houwink-Koeffizient des modifizierten Dendrimers mittels eines Ubelohde-Viskosimeters bestimmt. Der experimentelle Wert von α=0,211 kommt dem theoretischen Wert 0 für eine Kugelform der Makromoleküle in Lösung sehr nahe.

### Beispiel 2:

### Modifizierung von DAB(PA)₁₆ mit AEMA

15,46 g (0,02 mmol) DAG(PA)₁₆ (Fa. DSM, Dendrimer auf Polyethyleniminbasis mit 16 Aminoendgruppen) werden analog zu Beispiel 1 mit 58,94 (0,32 mol) AEMA umgesetzt. Es werden 77g (99%) einer klaren Flüssigkeit erhalten.

### Beispiel 3:

### Modifizierung von DAB(PA)₆₄ mit AEMA

21,51 g (3 mmol) DAB(PA)₆₄ (Fa. DSM, Dendrimer auf Polyethyleniminbasis mit 64 Aminoendgruppen) werden analog zu Beispiel 1 mit 63,66 g (384 mmol) AEMA umgesetzt. Es werden 83,4g (90%) einer klaren Flüssigkeit erhalten.

### Beispiel 4:

### Modifizierung von DAB(PA)₃₂ mit Allylacrylat

10,54 g (3 mmol) DAB(PA)₃₂ (Fa. DSM, Dendrimer auf Polyethyleniminbasis mit 32 Aminoendgruppen) werden analog zu Beispiel 1 mit 21,53 g (192 mmol) Allylacrylat in 5 ml Methylenchlorid umgesetzt. Nach Abdampfen des Lösungsmittels werden 28,7g (90%) einer öligen Flüssigkeit erhalten. 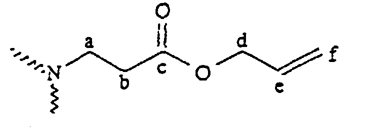 ¹H-NMR (ppm, CDCl₃, 90 MHz) :
4,57 d (OCH₂CH=), 5,8 m (CH=), 5,3 m (=CH₂).
¹³C-NMR (ppm, CDCl₃, 75 MHz) :
49,2 (a), 51,5 (b), 172,1 und 172,8 (c), 65,0 (d), 118,1 (e), 132,4 (f).
IR(Film, cm⁻¹) :
2948 (C-H), 1740 (C=O).

### Beispiel 5

### Modifizierung von DAB(PA)₃₂ mit AEMA und Ethylacrylat

10,54 g (3 mmol) DAB(PA)₃₂ (Fa. DSM) in 10 ml Methanol werden bei ca. 8°C unter Lichtausschluß tropfenweise mit einer Mischung aus 8,84g (48 mmol) AEMA und 14,24 g (144 mmol) Ethylacrylat in 5 ml Methanol versetzt (Molverhältnis AEMA : Ethylacrylat 1:3). Die Mischung wird eine Stunde bei Raumtemperatur und 24 Stunden bei 60°C gerührt, und anschließend wird das Methanol im Vakuum abgedampft. Es werden 33,5 g (99%) einer klaren Flüssigkeit erhalten. 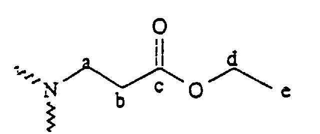 ¹H-NMR (ppm, CDCl₃, 90 MHz) :
1,3 (t, CH₃ Ethyl); 1,7 (m, CH₂CH₂CH₂); 2,0 (s, CH₃ Methacryl)
2,4-2,5 (CH₂-N); 2,8 (breites t, CH₂CH₂CO₂); 3,7 und 4,4 (O-CH₂CH₂-O) 4,2 (q, O-CH₂CH₃); 5,7 und 6,3; (=CH₂).
IR (Film, cm⁻¹) :
2952 (C-H), 1722(C=O), 1636(C=O), 1298, 1162(C-O).
¹³C-NMR(ppm, in CDCl₃, 75 MHz)
49,2(a), 51,5(b), 172,1 und 172,8(c), 65,0 (d), 118,1 (e), 132,4 (f), 51,8 (g).

### Beispiel 6

### Modizierung von DAB(PA)₆₄ mit AEMA und Trimethylsilylethylacrylat

10,75 g (1,5 mmol) DAB(PA)₆₄ (Fa. DSM, Dendrimer der 5. Generation auf Polyethyleniminbasis mit 64 Aminoendgruppen) in 5 ml Methanol werden tropfenweise mit einer Lösung von 7,07 g (38,4 mmol) AEMA und 26,47 g (153,6 mmol) 2-Trimethylsilylethylacrylat in 5 ml Methanol bei 8°C unter Lichtausschluß versetzt. Die Mischung wird 1 Stunde bei 8°C und 24 Stunden bei 60°C gerührt und anschließend wird das Reaktionsgemisch im Wasserstrahlvakuum bis zur Gewichtskonstanz eingeengt. Es werden 38,2g (86%) einer zähen Flüssigkeit erhalten. 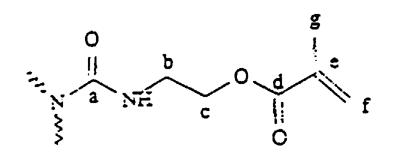 IR (KBr, cm⁻¹) :
3353 (N-H), 2949 (C-H), 1731 (C=O).
¹H-NMR (ppm, CDCl₃, 90 MHz) :
5,7 und 6,2 (2s, CH₂=), 4,1 (qu, OCH₂CH₃), 2,0 (s, CH₃ Methacr.), 1,2 (t, CH₂CH₃).
¹³C-NMR (ppm, CDCl₃, 75 MHz) :
172,4 (C=O, Ethylacrylat), 167,3 (C=O, Methacrylat),
158,3 (C=O, Harnstoff), 136,3 und
125,6 (C=CH₂).

### Beispiel 7:

### Modifizierung von DAB(PA)₈ mit 2-Isocyanatoethylmethylacrylat (IEM)

5,41 (7 mmol) DAB(PA)₈ (Fa. DSM, Dendrimer auf Polyethyleniminbasis mit 8 Aminoendgruppen) in 10 ml Methylenchlorid werden bei 8°C unter Lichtausschluß tropfenweise mit 8,41g (84 mmol) Ethylacrylat in 10 ml Methylenchlorid versetzt. Die Lösung wird eine Stunde bei 8 bis 10°C und 24 Stunden bei 50°C gerührt. Anschließend wird die Mischung bei 18 bis 22°C tropfenweise mit 4,34g (28 mmol) IEM (Polyscience) versetzt. Die Mischung wird für 5 Tage bei Raumtemperatur gerührt, und anschließend wird das Lösungsmittel im Vakuum abgedampft. Es werden 17 g (94%) einer klaren, viskosen Flüssigkeit erhalten.

### Beispiel 8:

### Umsetzung von DAB(PA)₈ mit Stearylacrylat

5,9 g (7,6 mmol) DAB(PA)₈ (Fa. DSM) in 10 ml Methylenchlorid werden bei 40°C zu 19,8 g (60 mmol) Stearylacrylat, das nach den üblichen Syntheseverfahren aus Stearylalkohol und Acrylsäurechlorid zugänglich ist, in 10 ml Methylenchlorid getropft. Die Mischung wird 24 Stunden bei 46 bis 48°C gerührt, und anschließend wird das Lösungsmittel im Vakuum abgedampft. Das Gefäß wird mit trockener Luft belüftet. Es werden 24,5g (95%) eines wachsähnlichen
Feststoffs erhalten (Fp.: 51 - 52°C).
IR (KBr,cm⁻¹) :
2916, 2850 (C-H), 1735 (C=O).
¹H-NMR (ppm, CDCl₃, 90 MHz):
1,3-1,5 (m, CH₂ der Stearyl-Kette), 1,0 (t, CH₃).
¹³C-NMR (ppm, CDCl₃, 75 MHz):
172,7 (C=O)

### Beispiel 9:

### Umsetzung von DAB(PA)₃₂ mit 2,2,3,4,4,4-Hexafluorbutylacrylat und AEMA

5,27g (1,5 mmol) DAB(PA)₃₂ (Fa. DSM) in 10 ml Methanol werden bei 8 bis 10°C tropfenweise mit einer Mischung aus 8,84 g (48 mmol) AEMA und 11,33 (48 mmol) 2,2,3,4,4,4-Hexafluorbutylacrylat (Fluorochem) in 10 ml Methanol versetzt. Die Lösung wird für eine Stunde bei 8 bis 10°C und für 24 Stunden bei 40°C gerührt. Anschließend wird das Reaktionsgemisch bei 30°C im Wasserstrahlvakuum bis zur Gewichtskonstanz eingeengt. Es werden 20,6g (81%) einer klaren, viskosen Flüssigkeit erhalten. 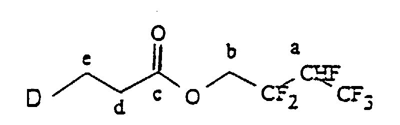 IR (KBr, cm⁻¹) :
2953, 2816 (C-H), 1736 (C=O).
¹H-NMR (ppm, CDCl₃, 90 MHz) :
4,6 (s, CHF), 3,8 (s, O-CH₂-CF₂).
¹³C-NMR (ppm, CDCl₃, 75 MHz):
172.7 (C=O).

### Beispiel 10:

### Homopolymerisation des modifizierten Dendrimeren aus Beispiel 1

Das Dendrimer gemäß Beispiel 1 wird unter Rühren mit einem Gewichtsprozent 2,2-Azo-bis-(2-methyl-propionsäurenitril) (AIBN) versetzt und so lange gerührt, bis eine homogene Lösung erhalten wird. Die Polymerisation wird mittels Differential Scanning Calorimetry (DSC) von 20 bis 170°C (Aufheizgeschwindigkeit: 10°C/min) verfolgt.

| | | |
|---|---|---|
| Ergebnisse | Polymerisationsenthalpie: | 49,8 kJ/mol |
| | Tg(Glasübergangstemperatur, Polymer): | -38,6°C |
| | Polymerisationsschrumpfung: | 7,3% |
| | (aus Dichtemessungen) | |

### Beispiel 11:

### Homopolymerisation des modifizierten Dendrimeren aus Beispiel 3

Das modifizierte Dendrimer aus Beispiel 3 wird unter Rühren mit 1 Gew.% AIBN versetzt und so lange gerührt, bis eine homogene Lösung erhalten wird. Die Polymerisation wird mittels DSC verfolgt.

| | | |
|---|---|---|
| Ergebnisse | Polymerisationsenthalpie: | 55,1kJ/ mol |
| | Tg(Polymer): | 40,9°C |
| | Polymerisationsschrumpfung: | 7,8% |
| | (aus Dichtemessungen) | |

### Beispiel 12:

### Herstellung von feinkörnigen Massen

39,2 Gew.% Bis-Phenol-A-glycidylmethacrylat, 20 Gew.% Urethandimethacrylat (aus 2,2,4-Trimethylhexamethylendiisocyanat und Hydroxyethylmethacrylat), 20 Gew.% Triethylenglykoldimethacrylat, 20 Gew.% modifiziertes Dendrimer gemäß Beispiel 2, 0,30 Gew.% Campherchinon und 0,50 Gew.% Cyanoethylmethylanilin (CEMA) werden in einer Knetmaschine (Fa. Linden) gemischt. 20 g dieser Monomermischung werden solange portionsweise mit silanisiertem Sphärosil (PALFI-QUE-S FILLER der Firma Tokuyama Soda, Japan) als Füllmittel versetzt, bis die Mischung Pastenkonsistenz aufweist (etwa 4-fache Gewichtsmenge der Monomermischung). Beim anschließenden Anlegen eines Vakuums (15 mbar) trocknet die Paste innerhalb weniger Sekunden und es entsteht ein feinkörniges Gemisch.

### Beispiel 13:

Analog Beispiel 12 wird eine feinkörnige Masse unter Verwendung von 20% modifiziertem Dendrimer gemäß Beispiel 1 und eines Gemischs aus silanisiertem feindispersen Siliciumdioxid und Ytterbium-Fluorid im Verhältnis von 67,3 zu 13,6 als Füllstoff hergestellt. Die Masse weist folgende Zusammensetzung auf: 18,33 Gew.% Monomergemisch (bestehend aus 39,2 Gew.% Bisphenol-A-Glycidylmethacrylat, 20 Gew.% Urethandimethacrylat (aus 2,2,4-Trimethylhexamethylendiisocyanat und Hydroxyethylmethacrylat), 20 Gew.% Triethylenglykoldimethacrylat, 20 Gew.% modifiziertes Dendrimer, 0,30 Campherchinon und 0,50 Gew.% CEMA), 67,12 Gew.% silanisiertes fein disperses Siliciumdioxid (Aerosil OX 50, Degussa AG), 14,55 Gew.% Ytterbiumfluorid. Der Dendrimergehalt der Gesamtmasse beträgt 7,2 Gew.%.

### Beispiel 14:

Analog Beispiel 13 wird eine feinkörnige Masse unter Verwendung von 20 Gew.% des Dendrimers gemäß Beispiel 3 hergestellt. Die Zusammensetzung der Masse entspricht der aus Beispiel 13.

### Beispiel 15:

Analog Beispiel 14 wird eine feinkörnige Masse jedoch ohne CEMA hergestellt, wobei im Vergleich zu Beispiel 14 als Ausgleich 0,5 Gew.-% Bisphenol-A-glycidylmethacrylat mehr verwendet werden.

### Beispiel 16 (Vergleichsbeispiel):

Analog Beispiel 14 wird eine fließfähige Masse ohne Dendrimer und ohne CEMA mit folgender Zusammensetzung hergestellt: 40,7 Gew.-% Bisphenol-A-glycidylmethacrylat, 39 Gew.-% Urethandimethacrylat aus 2-Hydroxyethylmethacrylat und 2,2,4-Trimethylhexamethylendiisocyanat-1,6, 20 Gew.-% Triethylenglycoldimethacrylat und 0,3 Gew.-% Campherchinon.

### Beispiel 17:

### Lichthärtung von körnigen Dendrimer-Kompositmassen

Das feinkörnige Komposit aus Beispiel 12 wird von Hand in Prüfkörperformen (2,5 mm x 2,0 mm x 2,0 mm) gepreßt, und die Prüfkörper werden anschließend gehärtet.

Nach der Härtung werden die Prüfkörper den Formen entnommen und einer 24stündigen Wasseranlagerung bei 37°C unterzogen (gemäß ISO-Norm 4049 (1988): Dentistry resin-based filling materials).

Die gehärteten Prüfkörper wurden anschließend auf ihre mechanischen Eigenschaften hin untersucht. Die Ergebnisse sowie die zur Härtung gewählten Bedingungen sind in den Tabellen I und II zusammengefaßt.

**Tabelle I**

| Mechanische Eigenschaften¹⁾ von Dentalkompositmassen gem. den Beispielen 13 und 14 | | |
|---|---|---|
| | Kompositmasse gem. Beispiel 14 | Kompositmasse gem. Beispiel 13 |
| Biegefestigkeit (N/mm²) nach Behandlung A | 59±7 | 51±7 |
| Biege-E-Modul (N/mm²) nach Behandlung A | 5700±500 | 5200±600 |
| Biegefestigkeit (N/mm²) nach Behandlung B | 101±12 | 96±19 |
| Biege-E-Modul (N/mm²) nach Behandlung B | 12000±2000 | 9400±1100 |
| Druckfestigkeit (N/mm²) nach Behandlung C | 176±28 | 205±35 |

| | | |
|---|---|---|
| ¹⁾ Bestimmt nach der ISO-Norm 4049 (1988) | | |

- Behandlung A:: 2 × 60 Sek. Bestrahlung mit einer Heliolux®-GTE-Lampe (Vivadent)
- Behandlung C:: 2 × 2 Sek. Bestrahlung mit einer Heliolux®-GTE-Lampe, 5 Min. Nachhärtung durch Belichtung mit einem Spectramat® (Ivoclar AG) bei gleichzeitiger Erwärmung auf 70°C.
- Behandlung C:: 3 × 60 Sek. Bestrahlung mit einer Heliolux®-GTE-Lampe

**Tabelle II**

| Lichtempfindlichkeit und Durchhärtungstiefe von Dentalkompositmassen mit metharylat-terminiertem Dendrimer im Vergleich zu einem herkömmlichen Komposit | | |
|---|---|---|
| | Kompositmasse gem. Beispiel 16 (Vergleich) | Kompositmasse gem. Beispiel 15 (Erfindung) |
| Durchhärtungstiefe¹⁾ | 4,6 mm | > 6 mm |
| Lichtempfindlichkeit¹) | 125 sec | 57 sec |
| Biegefestigkeit/Biege-E-Modul^{1,}³⁾ | 66±6 N/mm² 2400±200 N/mm² | 43±6 N/mm² 2400±380 N/mm² |
| Durchhärtungstiefe²⁾ | 3,4 mm | 5,4 mm |
| Lichtempfindlichkeit²⁾ | 125 sec | 70 sec |
| Biegefestigkeit/Biege-E-Modul^{2,}³⁾ | 19±2 N/mm² < 500 N/mm² | 32±11 N/mm² 250 N/mm² |

| | | |
|---|---|---|
| ¹⁾ ohne zusätzlichen Farbstoff | | |
| ²⁾ eingefärbt nach der Zahnfarbe 310 des Chromasscop®-Schlüssels, effektiver Farbstoffgehalt: 0,03 Gew.-% | | |
| ³⁾ bestimmt nach der ISO-Norm 4049 (1988) | | |

## Patentansprüche

1. Körnige, polymerisierbare Zusammensetzung, die mindestens ein polymerisierbares Monomer und/oder Oligomer sowie einen Polymerisationsinitiator und ggf. einen Beschleuniger und mindestens einen Füllstoff enthält, **dadurch gekennzeichnet**, daß sie mindestens 70 Gew.-% Füllstoff und zusätzlich 0,5 bis 28 Gew.-% Dendrimer enthält und bei Druck- und/oder Scherbeanspruchung fließfähig wird.

2. Polymerisierbare Zusammensetzung nach Anspruch 1, **dadurch gekennzeichnet**, daß das Dendrimer ein Propylenimin-, Polyether-/Polythioether-, Polyester-, Polyphenylenamid- und/oder Polyphenylenester-Dendrimer ist.

3. Polymerisierbare Zusammensetzung nach Anspruch 1 oder 2, **dadurch gekennzeichnet**, daß das Dendrimer polymerisationsfähige Endgruppen aufweist.

4. Polymerisierbare Zusammensetzung nach Anspruch 3, **dadurch gekennzeichnet**, daß das Dendrimer Meth(acryl)-, Allyl-, Styryl-, Vinyl-, Vinyloxy- und/oder Vinylamin-Endgruppen aufweist.

5. Polymerisierbare Zusammensetzung nach einem der Ansprüche 1 bis 4, **dadurch gekennzeichnet**, daß sie als polymerisierbares Monomer und/oder Oligomer ein oder mehrere mono- oder polyfunktionelle Methacrylate enthält.

6. Polymerisierbare Zusammensetzung nach einem der Ansprüche 1 bis 5, **dadurch gekennzeichnet**, daß sie 0,5 bis 28 Gew.-% mindestens eines polymerisierbaren Monomers und/oder Oligomers enthält.

7. Polymerisierbare Zusammensetzung nach einem der Ansprüche 1 bis 6, **dadurch gekennzeichnet**, daß sie als Füllstoff amorphe, pyrogene und/oder gefällte Kieselsäure mit einer BET-Oberfläche von 30 bis 300 m²/g, Zinkoxid (ZnO), röntgenopakes Glas, Bariumsulfat, Ytterbiumfluorid und/oder amorphe, kugelförmige Teilchen aus Siliciumdioxid mit bis zu 20 Mol-% mindestens eines Oxids eines Elements der Gruppen I, II, III und IV des Periodensystems mit einem Brechungsindex von 1,50 bis 1,58 und mit einer durchschnittlichen Primärteilchengröße von 0,1 bis 1,0 µm enthält.

8. Polymerisierbare Zusammensetzung nach einem der Ansprüche 1 bis 7, **dadurch gekennzeichnet**, daß sie bis zu 92 Gew.-% Füllstoff enthält.

9. Polymerisierbare Zusammensetzung nach einem der Ansprüche 1 bis 8, **dadurch gekennzeichnet**, daß sie als Initiator für die Photopolymerisation Benzophenon, Benzoin, ein Derivat von diesen oder ein α-Diketon und/oder als Initiator für die Kaltpolymerisation Benzoyl- oder Laurylperoxid zusammen mit einem Amin enthält.

10. Polymerisierbare Zusammensetzung nach Anspruch 9, **dadurch gekennzeichnet**, daß sie ein α-Diketon und zusätzlich ein Amin enthält.

11. Dentalmaterial, welches ein Gehalt an der polymerisierbaren Zusammensetzung gemäß einem der Ansprüche 1 bis 10 aufweist.

12. Verwendung der polymerisierbaren Zusammensetzung gemäß einem der Ansprüche 1 bis 10 als Dentalmaterial, Komponente eines Dentalmaterials oder zur Herstellung eines Dentalmaterials.

## Claims

1. Granular, polymerizable composition which comprises at least one polymerizable monomer and/or oligomer and a polymerization initiator and if appropriate an accelerator and at least one filler, characterized in that it comprises at least 70% by weight of filler and additionally 0.5 to 28% by weight of dendrimer and becomes flowable under compressive and/or shear stress.

2. Polymerizable composition according to Claim 1, characterized in that the dendrimer is a propyleneimine, polyether/polythioether, polyester, polyphenyleneamide and/or polyphenylene ester dendrimer.

3. Polymerizable composition according to Claim 1 or 2, characterized in that the dendrimer has polymerizable end groups.

4. Polymerizable composition according to Claim 3, characterized in that the dendrimer has meth(acryl), allyl, styryl, vinyl, vinyloxy and/or vinylamine end groups.

5. Polymerizable composition according to one of Claims 1 to 4, characterized in that it comprises one or more mono- or polyfunctional methacrylates as the polymerizable monomer and/or oligomer.

6. Polymerizable composition according to one of Claims 1 to 5, characterized in that it comprises 0.5 to 28% by weight of at least one polymerizable monomer and/or oligomer.

7. Polymerizable composition according to one of Claims 1 to 6, characterized in that it comprises, as a filler, amorphous, pyrogenic and/or precipitated silicic acid having a BET surface area of 30 to 300 m²/g, zinc oxide (ZnO), X-ray opaque glass, barium sulphate, ytterbium fluoride and/or amorphous, spherical particles of silicon dioxide with up to 20 mol% of at least one oxide of an element of groups I, II, III and IV of the periodic table with a refractive index of 1.50 to 1.58 and with an average primary particle size of 0.1 to 1.0 µm.

8. Polymerizable composition according to one of Claims 1 to 7, characterized in that it comprises up to 92% by weight of filler.

9. Polymerizable composition according to one of Claims 1 to 8, characterized in that it comprises, as an initiator for the photopolymerization, benzophenone, benzoin, a derivative of these or an α-diketone, and/or, as an initiator for the cold polymerization, benzoyl or lauryl peroxide, together with an amine.

10. Polymerizable composition according to Claim 9, characterized in that it comprises an α-diketone and additionally an amine.

11. Dental material which has a content of the polymerizable composition according to one of Claims 1 to 10.

12. Use of the polymerizable composition according to one of Claims 1 to 10 as a dental material, a component of a dental material or for the preparation of a dental material.

## Revendications

1. Composition polymérisable granulaire, contenant au moins un monomère et/ou un oligomère polymérisables ainsi qu'un initiateur de polymérisation et éventuellement un accélérateur et au moins une charge, caractérisée en ce qu'elle contient au moins 70 % en poids de charge et en outre 0,5 à 28 % en poids de dendrimère et est fluide sous contrainte de cisaillement et/ou de pression.

2. composition polymérisable selon la revendication 1, caractérisée en ce que le dendrimère est un dendrimère de type propylène-imine, polyéther/polythioéther, polyester, polyphénylène-amide et/ou polyphénylène-ester.

3. Composition polymérisable selon la revendication 1 ou 2, caractérisée en ce que le dendrimère comporte des groupes terminaux polymérisables.

4. Composition polymérisable selon la revendication 3, caractérisée en ce que le dendrimère comporte des groupes terminaux (méth)acryle, allyle, styryle, vinyle, vinyloxy et/ou vinylamino.

5. Composition polymérisable selon l'une des revendications 1 à 4, caractérisée en ce qu'elle contient en tant que monomère et/ou oligomère polymérisables un ou plusieurs méthacrylates mono- ou polyfonctionnels.

6. Composition polymérisable selon l'une des revendications 1 à 5, caractérisée en ce qu'elle contient de 0,5 à 28 % en poids d'au moins un monomère et/ou oligomère polymérisables.

7. Composition polymérisable selon l'une des revendications 1 à 6, caractérisée en ce qu'elle contient, en tant que charge, de l'acide silicique amorphe, pyrogène et/ou précipité ayant une surface BET de 30 à 300 m²/g, de l'oxyde de zinc (ZnO), du verre opaque aux rayons X, du sulfate de baryum, de fluorure d'ytterbium et/ou des particules sphériques amorphes de dioxyde de silicium comportant jusqu'à 20 % en moles d'au moins un oxyde d'un élément des groupes I, II, III et IV du tableau périodique, ayant un indice de réfraction de 1,50 à 1,58 et une taille moyenne de particules primaires de 0,1 à 1,0 µm.

8. Composition polymérisable selon l'une des revendications 1 à 7, caractérisée en ce qu'elle contient jusqu'à 92 % en poids de charge.

9. Composition polymérisable selon l'une des revendications 1 à 8, caractérisée en ce qu'elle contient, en tant qu'initiateur pour la photopolymérisation, de la benzophénone, de la benzoïne, un dérivé de celles-ci ou une α-dicétone et/ou, en tant qu'initiateur pour la polymérisation à froid, du peroxyde de benzoyle ou de lauryle, conjointement avec une amine.

10. Composition polymérisable selon la rerevendication 9, caractérisée en ce qu'elle contient une α-dicétone et en outre une amine.

11. Matériau dentaire, présentant une teneur en la composition polymérisable selon l'une des revendications 1 à 10.

12. Utilisation de la composition polymérisable selon l'une des revendications 1 à 10, en tant que matériau dentaire, composant d'un matériau dentaire ou pour la fabrication d'un matériau dentaire.
